# EUROPEAN PATENT SPECIFICATION

(11) **EP 3 996 800 B1**
(45) Date of publication and mention of the grant of the patent: **04.09.2024**
(21) Application number: 19745541.3
(22) Date of filing: 11.07.2019
(51) Int. Cl.: A61N 1/05, A61N 1/36

(54) **SYSTEMS FOR DETERMINING WHEN AN ELECTRODE LEAD REACHES A COCHLEAR BASAL TURN DURING A LEAD INSERTION PROCEDURE**
SYSTEME ZUR BESTIMMUNG, WANN EINE ELEKTRODENLEITUNG WÄHREND EINES LEITUNGSEINFÜHRUNGSVERFAHRENS EINE KOCHLEARE BASALWINDUNG ERREICHT
DES SYSTÈMES PERMETTANT DE DÉTERMINER QUAND UN CONDUCTEUR D'ÉLECTRODE ATTEINT UN TOUR BASAL COCHLÉAIRE PENDANT UNE PROCÉDURE D'INSERTION DE CONDUCTEUR

(43) Date of publication of application: 18.05.2022
(73) Proprietor: Advanced Bionics AG, 8712 Staefa (CH)
(72) Inventor: HUDAK, Eric M., Los Angeles, California 90012 (US); TARIGOPPULA, Venkata S. Aditya, Santa Clarita, California 91355 (US)
(74) Representative: Schwan Schorer & Partner mbB
(86) International application number: PCT/US2019/041303
(87) International publication number: WO 2021/006904

(56) References cited:
- WO-A1-2018/200450
- US-A- 5 674 264
- US-A1- 2018 280 687
- US-B1- 8 532 781

## Description

### BACKGROUND INFORMATION

During a lead insertion procedure in which a surgeon inserts an electrode lead into a cochlea of a recipient, a stylet is often used to hold the electrode lead and, in cases where the electrode array has a pre-curved shape, maintain the electrode lead in an initially straightened configuration. As the electrode lead approaches and passes the basal turn of the cochlea, the surgeon may release the stylet from the electrode lead and withdraw the stylet from the cochlea.

Recognizing when the distal end of the electrode lead is at the basal turn of the cochlea may enable the surgeon to release the stylet from the electrode lead at an optimal time, as well as to lessen the force applied during insertion, thus reducing a risk of insertion trauma and maximizing a possibility of correct electrode lead placement in the cochlea. Late stylet release can cause the electrode tip to touch or otherwise impact the lateral wall at the basal turn, which may cause trauma to the recipient, whereas premature stylet release may increase the possibility of a tip fold over and/or translocation of the electrode lead.

U.S. Patent 9,597,503 B2 ("Risi") discloses an intra-cochlear stimulating assembly insertion, and setting an angular insertion depth of a stimulating assembly during implantation into a recipient's cochlea. According to Risi, the angular insertion depth is monitored in real-time and advancement of the stimulating assembly is terminated when a selected angular insertion depth is achieved. Risi also discloses that a linear insertion depth that corresponds to a selected angular insertion depth is intraoperatively calculated and advancement of the stimulating assembly is terminated when the calculated linear insertion depth is achieved.

US 8,532,781 B1 relates to an example of monitoring of the position of an electrode lead of a cochlear implant when the electrode lead has been inserted into the cochlea, wherein a graphical representation of an intra-cochlear trajectory of electrodes is generated using electrical field imaging and wherein stimulation current is applied to one electrode and a resulting difference in impedance is recorded between two other electrodes. US 2018/0280687 A1 relates to a similar example, wherein differential measurements between electrodes are performed to determine a distance between the electrode array and a wall of the cochlea.

### BRIEF DESCRIPTION OF THE DRAWINGS

The accompanying drawings illustrate various embodiments and are a part of the specification. The illustrated embodiments are merely examples and do not limit the scope of the disclosure. Throughout the drawings, identical or similar reference numbers designate identical or similar elements.
FIG. 1 illustrates an exemplary cochlear implant system according to principles described herein.
FIG. 2 illustrates a schematic structure of the human cochlea according to principles described herein.
FIG. 3 illustrates an exemplary insertion management system according to principles described herein.
FIG. 4 illustrates an exemplary implementation of the insertion management system of FIG. 3 according to principles described herein.
FIGS. 5A-5C illustrate an exemplary insertion procedure in which an electrode lead is inserted into a cochlea of a recipient according to principles described herein.
FIGS. 6A-6C illustrate various electrode stimulating and recording electrode configurations that may be used according to principles of the present disclosure.
FIG. 7 shows a simplified representation of an exemplary graph of response signals of a cochlear electrode lead during an insertion procedure according to principles of the present disclosure.
FIG. 8 illustrates an exemplary graphical user interface that may be displayed by a display device at the direction of an insertion management system according to embodiments of the present disclosure.
FIGS. 9-10 illustrate exemplary methods according to principles described herein.
FIG. 11 illustrates an exemplary computing device according to principles described herein.

### DETAILED DESCRIPTION

The invention relates to a system as defined in claim 1 for determining when an electrode lead reaches a cochlear basal turn during a lead insertion procedure. For example, as described herein, an insertion management system directs a cochlear implant to apply electrical stimulation by way of a first electrode disposed on an electrode lead that is being inserted into a cochlea of a recipient. While the electrical stimulation is being applied by way of the first electrode, the insertion management system directs the cochlear implant to record a differential voltage signal representative of a differential voltage between a second electrode and a third electrode both disposed on the electrode lead and different than the first electrode. While the differential voltage signal is being recorded, the insertion management system determines that a signal characteristic of the differential voltage signal changes by at least a threshold amount. Based on the signal characteristic changing by at least the threshold amount, the insertion management system determines that a distal end of the electrode lead is positioned within a threshold distance of a basal turn of the cochlea.

The systems described herein may be used in connection with any suitable type of electrode lead. For example, the systems and methods described herein may be used in connection with an electrode lead that has a naturally pre-curved shape. This type of electrode lead may be referred to as a perimodiolar electrode lead and may be configured to hug a modiolar wall of a cochlea when inserted within the cochlea. The systems described herein may additionally or alternatively be used in connection with an electrode lead that has a naturally straight shape. This type of electrode lead may be referred to as a mid-scalar electrode lead, and may be configured to conform to the shape of the cochlea as the electrode lead is inserted into the cochlea.

The systems described herein may advantageously provide real-time feedback regarding a positioning of an electrode lead within the cochlea during a lead insertion procedure. This real-time feedback may optimize timing of stylet release and removal from an electrode lead being inserted into the cochlea, thereby reducing a risk of insertion trauma (e.g., trauma caused by scraping, piercing, irritating, and/or otherwise damaging a wall of the cochlea), maximizing a possibility of correct electrode lead placement in the cochlea, preventing tip-foldover, and providing an optimal insertion trajectory for the electrode lead into the cochlea. These and other advantages and benefits of the systems described herein will be made apparent herein.

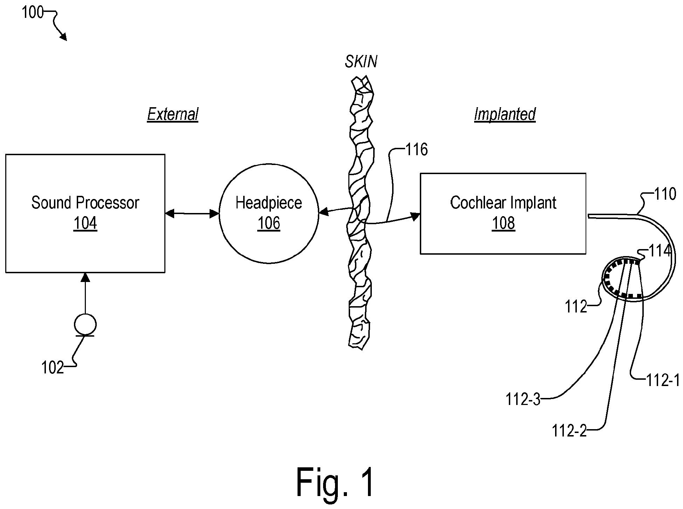

FIG. 1 illustrates an exemplary cochlear implant system 100: As shown, cochlear implant system 100 may include a microphone 102, a sound processor 104, a headpiece 106 having a coil disposed therein, a cochlear implant 108, and an electrode lead 110. Electrode lead 110 may include an array of electrodes 112 disposed on a distal portion of electrode lead 110 and that are configured to be inserted into a cochlea of a recipient to stimulate the cochlea when the distal portion of electrode lead 110 is inserted into the cochlea. One or more other electrodes (e.g., including a ground electrode, not explicitly shown) may also be disposed on other parts of electrode lead 110 (e.g., on a proximal portion of electrode lead 110) to, for example, provide a current return path for stimulation current generated by electrodes 112 and to remain external to the cochlea after electrode lead 110 is inserted into the cochlea. As described herein, electrode lead 110 may be naturally pre-curved or straight. Additional or alternative components may be included within cochlear implant system 100 as may serve a particular implementation.

As shown, cochlear implant system 100 may include various components configured to be located external to a recipient including, but not limited to, microphone 102, sound processor 104, and headpiece 106. Cochlear implant system 100 may further include various components configured to be implanted within the recipient including, but not limited to, cochlear implant 108 and electrode lead 110.

Microphone 102 may be configured to detect audio signals presented to the user. Microphone 102 may be implemented in any suitable manner. For example, microphone 102 may include a microphone that is configured to be placed within the concha of the ear near the entrance to the ear canal, such as a T-MIC^{™} microphone from Advanced Bionics. Such a microphone may be held within the concha of the ear near the entrance of the ear canal during normal operation by a boom or stalk that is attached to an ear hook configured to be selectively attached to sound processor 104. Additionally or alternatively, microphone 102 may be implemented by one or more microphones disposed within headpiece 106, one or more microphones disposed within sound processor 104, one or more beam-forming microphones, and/or any other suitable microphone as may serve a particular implementation.

Sound processor 104 may be housed within any suitable housing (e.g., a behind-the-ear ("BTE") unit, a body worn device, headpiece 106, and/or any other sound processing unit as may serve a particular implementation). Sound processor 104 may be configured to direct cochlear implant 108 to generate and apply electrical stimulation (e.g., a sequence of stimulation pulses) by way of one or more electrodes 112.

As shown, electrodes 112 may include a first electrode 112-1 that is most distally positioned on electrode lead 110 (i.e., closest to a distal end 114 of electrode lead 110), a second electrode 112-2 adjacent to electrode 110-1, a third electrode 112-3 adjacent to electrode 112-2, etc. Any number of electrodes 112 (e.g., sixteen) may be disposed on electrode lead 110 as may serve a particular implementation. While electrodes 112 are shown as linearly positioned along a side of electrode lead 110 that faces a modiolar wall of cochlea 200, other configurations for electrodes 112 may be implemented. For example, diametrically opposed electrodes 112 may be implemented (e.g., a first electrode and a second electrode may be positioned diametrically to one another, while a third and fourth electrode may be positioned diametrically to one another and linearly following first and second electrodes). As another example, the distal-most electrode may be a tip electrode (i.e., located a distal end 114 of electrode 110).

In some examples, sound processor 104 may direct cochlear implant 108 to apply electrical stimulation representative of an audio signal presented to the recipient. The audio signal may be detected by microphone 102, input by way of an auxiliary audio input port, input by way of a clinician's programming interface (CPI) device, and/or otherwise provided to sound processor 104. Sound processor 104 may process the audio signal in accordance with a selected sound processing strategy or program to generate appropriate stimulation parameters for controlling cochlear implant 108.

Additionally or alternatively, sound processor 104 (and/or any other suitable computing device) may direct cochlear implant 108 to generate and apply electrical stimulation that is not representative of audio signals to the patient. For example, as described herein, sound processor 104 and/or any other suitable computing device may direct cochlear implant 108 to apply a sequence of stimulation pulses by way of one or more electrodes 112 during a lead insertion procedure.

In some examples, sound processor 104 may wirelessly transmit stimulation parameters (e.g., in the form of data words included in a forward telemetry sequence) and/or power signals to cochlear implant 108 by way of a wireless communication link 116 between headpiece 106 and cochlear implant 108 (e.g., a wireless link between a coil disposed within headpiece 106 and a coil physically coupled to cochlear implant 108). It will be understood that communication link 116 may include a bi-directional communication link and/or one or more dedicated uni-directional communication links.

Headpiece 106 may be communicatively coupled to sound processor 104 and may include an external antenna (e.g., a coil and/or one or more wireless communication components) configured to facilitate selective wireless coupling of sound processor 104 to cochlear implant 108. Headpiece 106 may additionally or alternatively be used to selectively and wirelessly couple any other external device to cochlear implant 108. To this end, headpiece 106 may be configured to be affixed to the recipient's head and positioned such that the external antenna housed within headpiece 106 is communicatively coupled to a corresponding implantable antenna (which may also be implemented by a coil and/or one or more wireless communication components) included within or otherwise associated with cochlear implant 108. In this manner, stimulation parameters and/or power signals may be wirelessly transmitted between sound processor 104 and cochlear implant 108 via communication link 116.

Cochlear implant 108 may include any suitable type of implantable stimulator. For example, cochlear implant 108 may be implemented by an implantable cochlear stimulator. Additionally or alternatively, cochlear implant 108 may include a brainstem implant and/or any other type of cochlear implant that may be implanted within a recipient and configured to apply stimulation to one or more stimulation sites located along an auditory pathway of a recipient.

In some examples, cochlear implant 108 may be configured to generate electrical stimulation in accordance with one or more stimulation parameters transmitted thereto by sound processor 104. Cochlear implant 108 may be further configured to apply the electrical stimulation to one or more electrodes 112 disposed along electrode lead 110. Such stimulation may be configured as monopolar stimulation (e.g., stimulation applied by way of a single electrode 112 with a remote electrode on a case of cochlear implant 108 or on a proximal portion of electrode lead 108 being used as a return electrode) or as multipolar stimulation (e.g., bipolar stimulation applied via by way of two electrodes 112). In some examples, cochlear implant 108 may include a plurality of independent current sources each associated with a channel defined by one or more of electrodes 112. In this manner, different stimulation current levels may be applied to multiple stimulation sites simultaneously by way of multiple electrodes 112.

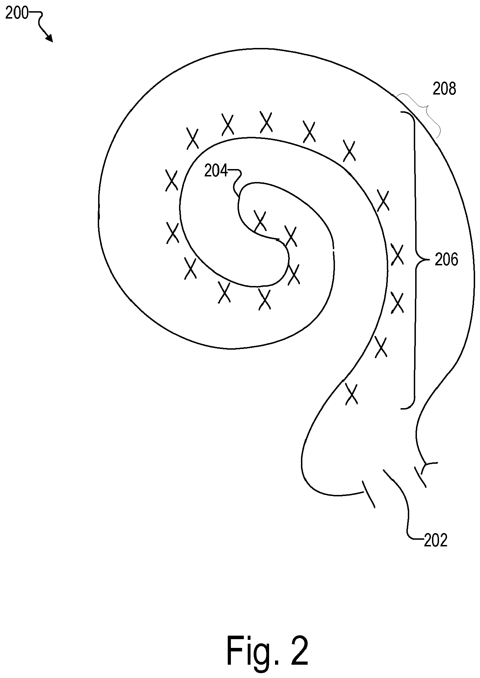

FIG. 2 illustrates a schematic structure of the human cochlea 200 into which electrode lead 110 may be inserted. As shown in FIG. 2, cochlea 200 is in the shape of a spiral beginning at a base 202 and ending at an apex 204. Within cochlea 200 resides auditory nerve tissue, which is denoted by Xs in FIG. 2. The auditory nerve tissue is organized within the cochlea 200 in a tonotopic manner. Relatively low frequencies are encoded at or near the apex 204 of the cochlea 200 referred to as an "apical region" while relatively high frequencies are encoded at or near the base 202 in a basal region 206 of the cochlea 200.

FIG. 2 illustrates a region of cochlea 200 referred to as a basal turn 208. As shown, basal turn 208 is located at an end of basal region 206. As described herein, the systems are configured to determine, in real-time during a lead insertion procedure, when distal end 114 of electrode lead 110 is within a threshold distance of basal turn 208 (e.g., when distal end 114 of electrode lead 110 is approaching but not yet at basal turn 208, at basal turn 208, or slightly past basal turn 208). In so doing, it may be possible for an operator to begin removal of a stylet holding electrode lead 110 in a straightened configuration, as well as to avoid damage (e.g., scraping or puncture) of cochlea 200 by electrode lead 110 and/or the stylet.

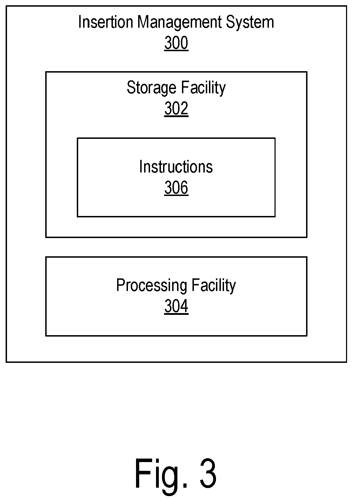

FIG. 3 illustrates an exemplary insertion management system 300 ("system 300") that may be configured to perform any of the operations described herein. As shown, system 300 may include, without limitation, a storage facility 302 and a processing facility 304 selectively and communicatively coupled to one another. Facilities 302 and 304 may each include or be implemented by hardware and/or software components (e.g., processors, memories, communication interfaces, instructions stored in memory for execution by the processors, etc.). In some examples, facilities 302 and 304 may be distributed between multiple devices and/or multiple locations as may serve a particular implementation.

Storage facility 302 may maintain (e.g., store) executable data used by processing facility 304 to perform any of the operations described herein. For example, storage facility 302 may store instructions 306 that may be executed by processing facility 304 to perform any of the operations described herein. instructions 306 may be implemented by any suitable application, software, code, and/or other executable data instance. Storage facility 302 may also maintain any data received, generated, managed, used, and/or transmitted by processing facility 304.

Processing facility 304 may be configured to perform (e.g., execute instructions 306 stored in storage facility 302 to perform) various operations associated with determining when an electrode lead (e.g., electrode lead 110) reaches a basal turn (e.g., basal turn 208) of a cochlea (e.g., cochlea 200) during an electrode lead insertion procedure in which the electrode lead is inserted into the cochlea. For example, processing facility 304 may direct a cochlear implant (e.g., cochlear implant 108) to apply electrical stimulation by way of a first electrode (e.g., electrode 112-2) disposed on the electrode lead while the electrode lead is being inserted into a cochlea of a recipient. While the electrical stimulation is being applied by way of the first electrode, processing facility 304 may direct the cochlear implant to record a differential voltage signal between a second electrode (e.g., electrode 112-1) and a third electrode (e.g., electrode 112-3) disposed on the electrode lead. While the differential voltage signal is being recorded, processing facility 304 may determine that a signal characteristic of the differential voltage signal changes by at least a threshold amount. Processing facility 304 may determine, based on the signal characteristic changing by at least the threshold amount, that a distal end 114 of the electrode lead is positioned within a threshold distance of a basal turn of the cochlea. These and other operations that may be performed by processing facility 304 (i.e., by system 300) are described in more detail herein.

System 300 may be implemented in any suitable manner. For example, system 300 may be implemented entirely by sound processor 104. Alternatively, system 300 may be implemented entirely by a computing device configured to be communicatively coupled to sound processor 104. Alternatively, system 300 may be implemented by both sound processor 104 and a computing device configured to be communicatively coupled to sound processor 104.

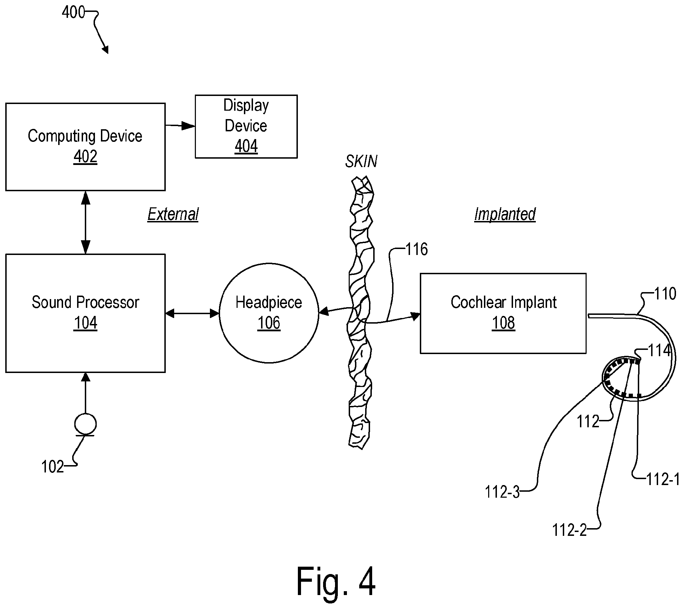

FIG. 4 shows an exemplary configuration 400 in which system 300 is at least partially implemented by a computing device 402 configured to communicatively couple to sound processor 104. In some examples, computing device 402 may be configured to direct cochlear implant 108 to generate and apply electrical stimulation by way of one or more electrodes 112.

Computing device 402 may be implemented by any suitable combination of hardware (e.g., one or more computing devices) and software. To illustrate, computing device 402 may be implemented by a desktop computer, a mobile device (e.g., a laptop, a smartphone, a tablet computer, etc.), and/or any other suitable computing device as may serve a particular implementation. In some examples, computing device 402 is included in a fitting system configured to be used to fit cochlear implant system 100 to a recipient.

As shown, computing device 402 is communicatively coupled to a display device 404. While display device 404 is illustrated to be separate from computing device 402 in FIG. 4, display device 404 may alternatively be integrated into computing device 402. Display device 404 may be implemented by any suitable device configured to display graphical content generated by computing device 402. For example, display device 404 may display one or more graphs of signals recorded by electrodes 112 disposed on electrode lead 110.

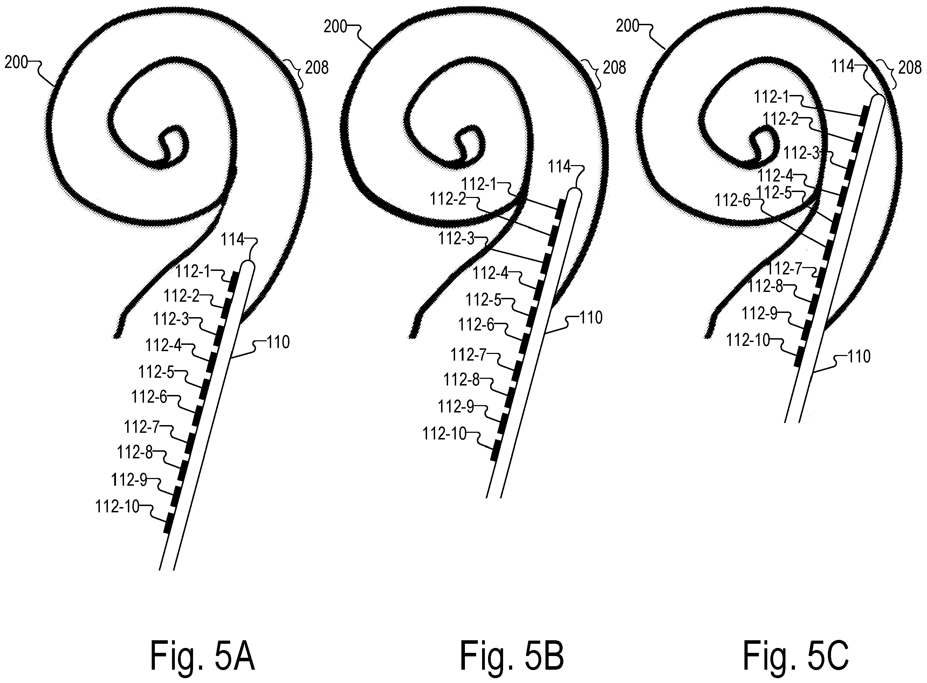

FIGS. 5A-5C illustrate an exemplary insertion procedure in which electrode lead 110 of the cochlear implant 108 is inserted into a cochlea 200 of a recipient according to principles of the present disclosure. Electrode 112-1 is a distal-most electrode on lead 110 located closest to distal end 114 of lead 110. The number of electrodes 112 shown as being disposed on lead 110 is exemplary only, and has been limited to simplify the following discussion. Any suitable number of electrodes 112 greater than or equal to 3 may be implemented on lead 110 without departing from the scope of the present disclosure. Moreover, although the electrodes 112 are sequentially numbered, proceeding linearly along a length of lead 110, the term "a first electrode" does not necessarily mean electrode 112-1, absent indication to the contrary, and the reference symbols are intended as referential, cardinal numbers.

FIG. 5A shows electrode lead 110 entering cochlea 200. In this figure, electrodes 112-1 and 112-2 have barely entered cochlea 200, while electrodes 112-3 to 112-10 remain outside cochlea 200. FIG. 5B shows electrode lead 110 after electrode lead 110 has been advanced further into cochlea 200 such that electrodes 112-1 to electrodes 112-4 are positioned within cochlea 200, while electrodes 112-5 to 112-10 (not labeled in FIG. 5B) remain outside of cochlea 200. As shown in FIG. 5B, a distal end 114 of lead 110, while nearing the basal turn of the cochlea 208, has not yet arrived at this point.

FIG. 5C shows electrode lead 110 when electrode lead 110 has been advanced further into cochlea 200 such that distal end 114 of electrode lead 110 is positioned within a threshold distance of basal turn 208 of cochlea 200. The threshold distance may be, for example, less than three millimeters or any other suitable distance. In some examples, the threshold distance may be set in response to user input, determined automatically based on one or more characteristics of the recipient (e.g., a pediatric recipient may be associated with a smaller threshold distance than an adult recipient), and/or determined in any other manner.

An exemplary manner in which system 300 may determine that distal end 114 is within the threshold distance of basal turn 208 will now be described with reference to FIGS. 5A-5C.

As electrode lead 110 is being inserted into cochlea 200, system 300 may direct cochlear implant 108 to apply electrical stimulation by way of at least one electrode. The electrical stimulation may include, for example, a sequence of stimulation pulses applied at any suitable stimulation frequency. The stimulation pulses may have any suitable fixed current level as may serve a particular implementation.

In some examples, the electrical stimulation is monopolar. In these examples, the electrical stimulation is applied to only one of electrodes 112, with a remote electrode (e.g., an electrode disposed on a case of cochlear implant 108 or on a proximal portion of electrode lead 108) being used as a return electrode for the electrical stimulation. The single electrode 112 by way of which the monopolar stimulation is applied may be any one of electrodes 112, but in many implementations, the single electrode is located more toward distal end 114 of electrode lead 110 (e.g., electrode 112-1, electrode 112-2, or electrode 112-3).

In alternative examples, the electrical stimulation is bipolar. In these examples, the electrical stimulation is applied by way of two of electrodes 112, with one of the two electrodes serving as the return electrode. For example, bipolar stimulation may be applied by way of electrodes 112-2 and 112-3, electrodes 112-1 and 112-4, or any other suitable combination of electrodes.

As used herein, the term "stimulating electrodes" refers to electrodes used to apply electrical stimulation. While the electrical stimulation is being applied by way of the stimulating electrode(s), system 300 may direct cochlear implant 108 to record a differential voltage signal representative of a differential voltage between two electrodes that are different than the stimulating electrodes. These electrodes used to record the differential voltage signal are referred to as "recording electrodes" herein. The two recording electrodes may be any combination of electrodes other than the stimulating electrodes. For example, if electrodes 112-2 and 112-3 serve as the stimulating electrodes, electrodes 112-1 and 112-4 may serve as the recording electrodes. As another example, if 112-1 and 112-4 serve as the stimulating electrodes, electrodes 112-2 and 112-3 may serve as the recording electrodes. In some implementations, the distal-most electrode 112-1 has to serve as either a stimulating electrode or as a recording electrode.

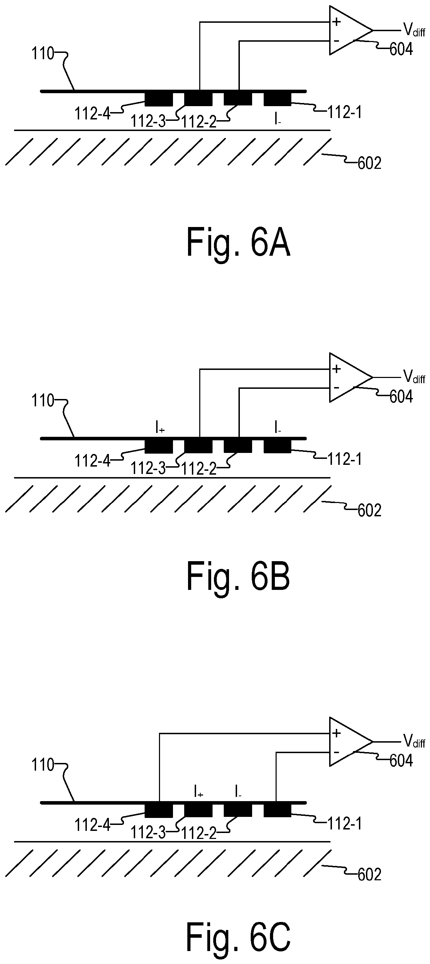

FIGS. 6A-6C illustrate various stimulating and recording electrode configurations that may be used in connection with the systems described herein. In each of the configurations shown in FIGS. 6A-6C, electrode lead 110 is positioned within cochlea 200 adjacent to cochlear tissue 602. It will be recognized that configurations other than those illustrated in FIGS. 6A-6C may also be used in connection with the systems described herein

In FIG. 6A, distal-most electrode 112-1 on electrode lead 110 is configured to be a stimulating electrode and electrodes 112-2 and 112-3 on electrode lead 110 are configured to be recording electrodes. In this configuration, monopolar stimulation is applied by way of electrode 112-1 while a differential voltage measuring circuit 604 uses electrodes 112-2 and 112-3 to record a differential voltage between electrodes 112-2 and 112-3. Differential voltage measuring circuit 604 may be implemented by any suitable combination of components in cochlear implant 110 and/or sound processor 104.

In FIG. 6B, distal-most electrode 112-1 and electrode 112-4 are configured to be stimulating electrodes and electrodes 112-2 and 112-3 are configured to be recording electrodes. In this configuration, bipolar stimulation is applied by way of electrodes 112-1 and 112-4 while differential voltage measuring circuit 604 uses electrodes 112-2 and 112-3 to record a differential voltage between electrodes 112-2 and 112-3.

In FIG. 6C, electrodes 112-2 and 112-3 are configured to be stimulating electrodes and electrodes 112-1 and electrode 112-4 are configured to be recording electrodes. In this configuration, bipolar stimulation is applied by way of electrodes 112-2 and 112-3 while differential voltage measuring circuit 604 uses electrodes 112-1 and 112-2 to record a differential voltage between electrodes 112-1 and 112-2.

While the differential voltage is being recorded, system 300 may determine that a signal characteristic of the differential voltage signal changes by at least a threshold amount. Based on the signal characteristic changing by at least the threshold amount, system 300 may determine that distal end 114 is positioned within the threshold distance of basal turn 208 of cochlea 200.

The signal characteristic of the differential voltage signal may include any characteristic that may be indicative of an arrival of distal end 114 of electrode lead 110 at basal turn 208. For example, the signal characteristic may be a slope associated with the differential voltage signal, a rate of change associated with the differential voltage signal, an amplitude associated with the differential voltage signal, and/or any other suitable characteristic associated with the differential voltage signal as may serve a particular implementation.

System 300 may determine the signal characteristic of the differential voltage signal in any suitable manner. For example, system 300 may process the differential voltage signal itself to determine the signal characteristic. Additionally or alternatively, system 300 may generate an impedance signal based on the differential voltage signal and then identify the signal characteristic based on the impedance signal. The impedance signal may be generated by, for example, dividing the differential voltage signal by the amplitude of the electrical stimulation in accordance with Ohm's law.

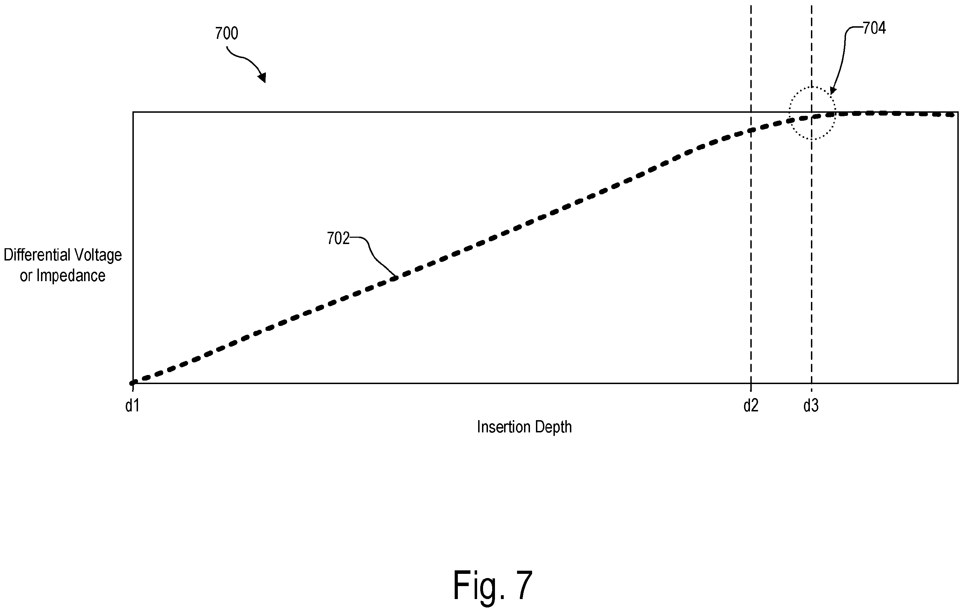

FIG. 7 shows an exemplary graph 700 of a signal 702 that may be representative of either a differential voltage signal or an impedance signal generated based on the differential voltage signal, and shows how an amplitude of signal 702 may increase as an insertion depth of electrode lead 110 increases during a lead insertion procedure in which electrode lead 110 is inserted into cochlea 200. As shown, a slope of signal 702 remains relatively constant between insertion depth d1 and insertion depth d2. At insertion depth d2, the slope of signal 702 begins to decrease until it reaches a point of inflection 704 at insertion depth d3. After insertion depth d3, the slope goes slightly negative.

This change in slope correlates to when distal end 114 of lead 110 is within a threshold distance of basal turn 208. Hence, system 300 may track the slope of signal 702 and determine, when the slope changes by at least a threshold amount, that distal end 114 of lead 110 is within a threshold distance of basal turn 208. Other signal characteristics of signal 702 may be similarly tracked and used to determine when distal end 114 of lead 110 is within a threshold distance of basal turn 208.

It will be recognized that graph 700 is merely illustrative and that signal 702 may have different characteristics compared to those shown in FIG. 7 (e.g., different slopes, shapes, etc.). For example, insertion of a naturally pre-curved electrode lead may result in a signal similar to signal 702 that peaks at an insertion depth that corresponds to when a distal end of the electrode lead is within the predetermined threshold of basal turn 208. However, insertion of a naturally straight electrode lead may result in a signal that may not peak. In this case, the slope of the signal may still decrease by a certain amount when a distal end of the electrode lead is within the predetermined threshold of basal turn 208. Hence, in some examples, the threshold amount by which the slope (and/or any other signal characteristic of signal 702) has to change may be based on the type of electrode lead being used, one or more characteristics of the recipient, a current level of the electrical stimulation being used, etc.

Data representative of the threshold amount and/or threshold distance may be accessed by system 300 in any suitable manner. In some examples, system 300 may store data representative of the threshold amount and/or threshold distance in storage facility 302, access such data stored remotely from system 300 (e.g., at a server connected to system 300 by way of a network), etc.

System 300 may perform various operations based on and/or in response to the determination that distal end 114 of electrode lead 110 is within the threshold distance of basal turn 208 of cochlea 200. For example, system 300 may provide a notification (e.g., an alert) in substantially real-time that distal end 114 is within the threshold distance. As another example, in response to determining that distal end 114 of electrode lead 110 is within the threshold distance of basal turn 208 of cochlea 200, system 300 may transmit one or more commands to a surgeon and/or a tool being used to perform the lead insertion procedure to release a stylet from electrode lead 110, retract the stylet from the cochlea 200, slow down an insertion rate of electrode lead 110 into cochlea 200, and/or perform any other suitable operation as may serve a particular implementation.

In some examples, system 300 may plot, within an interface displayed on a display device, a graph associated with the differential voltage signal in substantially real time as the differential voltage signal is being recorded. In this manner, a user (e.g., a surgeon) may visually ascertain when electrode lead 110 is nearing basal turn 208.

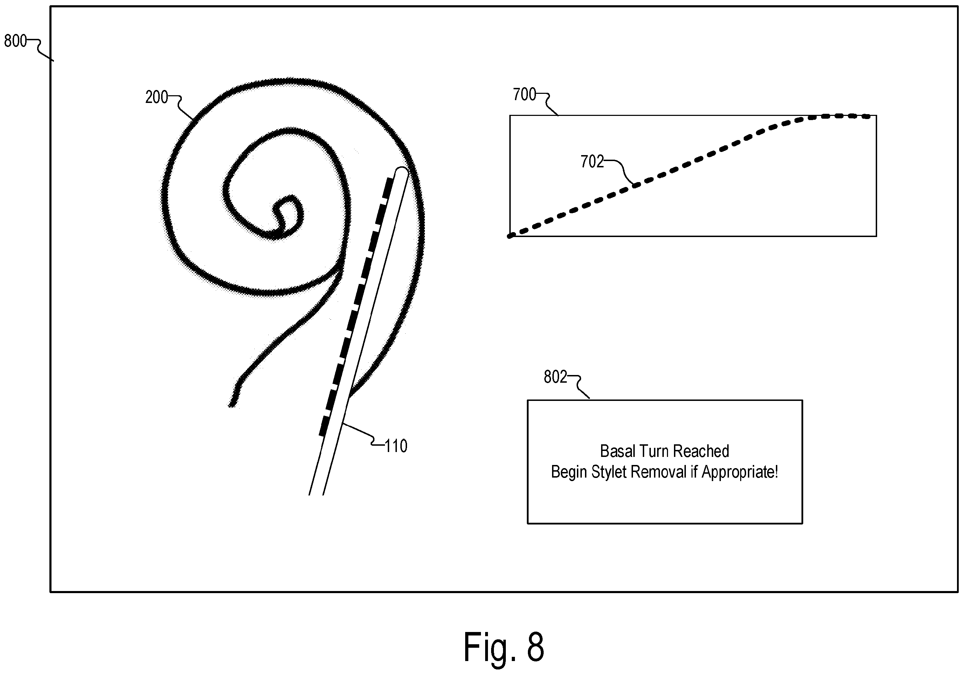

To illustrate, FIG. 8 illustrates an exemplary graphical user interface 800 that may be displayed by a display device (e.g., display device 404) at the direction of system 300 according to embodiments of the present disclosure. As shown, graphical user interface 800 may include a graphical representation of graph 700. In some examples, signal 702 is plotted in substantially real time as the differential voltage signal is being recorded.

In some examples, system 300 may additionally or alternatively present, within interface 800, a progress indicator indicating a location of electrode lead 110 within cochlea 200 based on the signal characteristic being monitored by system 300. For example, as shown in FIG. 8, graphical user interface 800 may include a graphical representation of cochlea 200 that shows a graphical representation of electrode lead 110 being inserted into cochlea 200. These graphical representations may serve as the progress indicator indicating a location of electrode lead 110 within cochlea 200.

As mentioned, system 300 may provide a notification in response to determining that distal end 114 of electrode lead 110 has reached basal turn 208. To illustrate, FIG. 8 shows that a graphical notification 802 may be presented within graphical user interface 800. System 300 may additionally or alternatively provide an audible notification and/or any other type of notification as may serve a particular implementation.

In some examples, the real-time feedback provided during a lead insertion procedure as described herein may be used by system 300 to perform one or more other types of operations. For example, the real-time feedback provided by system 300 may be used by system 300 to determine a trajectory of insertion of electrode lead 110 into cochlea 200 until distal end 114 of electrode lead 110 reaches basal turn 208. For example, based on a rate of change of a slope of signal 702, a distance covered and/or time elapsed without a significant change in slope of signal 702, and/or any other characteristic of signal 702, system 300 may determine the trajectory of insertion and provide real-time feedback (e.g., by way of graphical user interface 800) representative of the trajectory of insertion of lead 110. This may be used by a user (e.g., a surgeon) to modify the trajectory of insertion as needed and/or desired.

In some examples, system 300 may additionally or alternatively determine, based on a signal characteristic of signal 702 changing by at least the threshold amount, a distance between a round window of a recipient and the basal turn of the recipient. For example, system 300 may use any suitable technique to determine a distance between d1 and d3 shown in FIG. 7 and designate this distance as the distance between the round window of the recipient and the basal turn of the recipient. Data representative of this distance may be used by system 300 to set one or more stimulation parameters for cochlear implant system 100 and/or for any other reason as may serve a particular implementation.

In some examples, once system 300 has determined that distal end 114 of electrode lead 110 has reached basal turn 208, system 300 may switch to a new set of stimulating and recording electrodes in order to ascertain which and/or how many electrodes have passed basal turn 208 during the lead insertion procedure. For example, with references to FIGS. 5A-5C, system 300 may initially designate electrodes 112-2 and 112-3 as stimulating electrodes and electrodes 112-1 and 112-4 as recording electrodes. Once, distal end 114 of electrode lead 110 reaches basal turn 208, system 300 may stop using electrodes 112-1 through 112-4 as stimulating and recording electrodes and instead use electrodes 112-6 and 112-7 as stimulating electrodes and electrodes 112-5 and 112-8 as recording electrodes. In this manner, system 300 may use the techniques described herein to determine when one or more of these electrodes (e.g., individual electrodes and/or groupings of multiple electrodes, such as groupings of three or four electrodes) are within the threshold distance of basal turn 208. System 300 may provide real-time feedback regarding how many electrodes have passed basal turn 208 in any suitable manner (e.g., by way of graphical user interface 800).

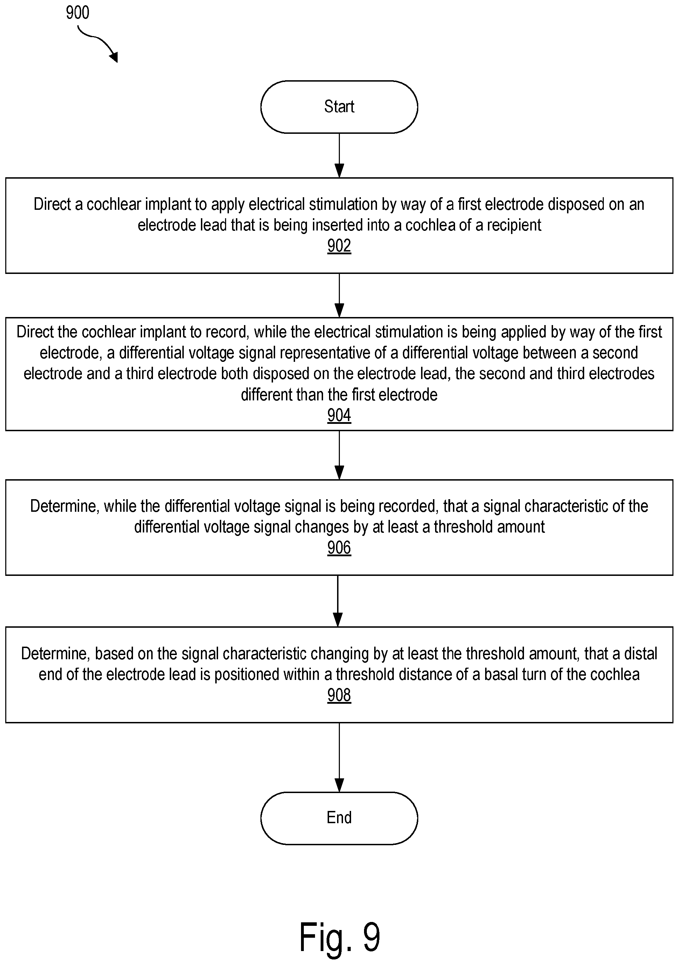

FIG. 9 illustrates an exemplary method according to principles described herein. The operations shown in FIG. 9 may be performed by system 300 and/or any implementation thereof. While FIG. 9 illustrates exemplary operations according to one embodiment, other embodiments may omit, add to, reorder, and/or modify any of the operations shown in FIG. 9.

In operation 902, an insertion management system directs cochlear implant to apply electrical stimulation by way of a first electrode disposed on an electrode lead that is being inserted into a cochlea of a recipient. Operation 902 may be performed in any of the ways described herein.

In operation 904, the insertion management system directs the cochlear implant to record, while the electrical stimulation is being applied by way of the first electrode, a differential voltage signal representative of a differential voltage between a second electrode and a third electrode, both being disposed on the electrode lead, and the second and third electrodes different than the first electrode. Operation 904 may be performed in any of the ways described herein.

In operation 906, the insertion management system determines, while the differential voltage signal is being recorded, that a signal characteristic of the differential voltage signal changes by at least a threshold amount. Operation 906 may be performed in any of the ways described herein.

In operation 908, the insertion management system determines, based on the signal characteristic changing by at least the threshold amount, that a distal end 114 of the electrode lead is positioned within a threshold distance of a basal turn of the cochlea. Operation 908 may be performed in any of the ways described herein.

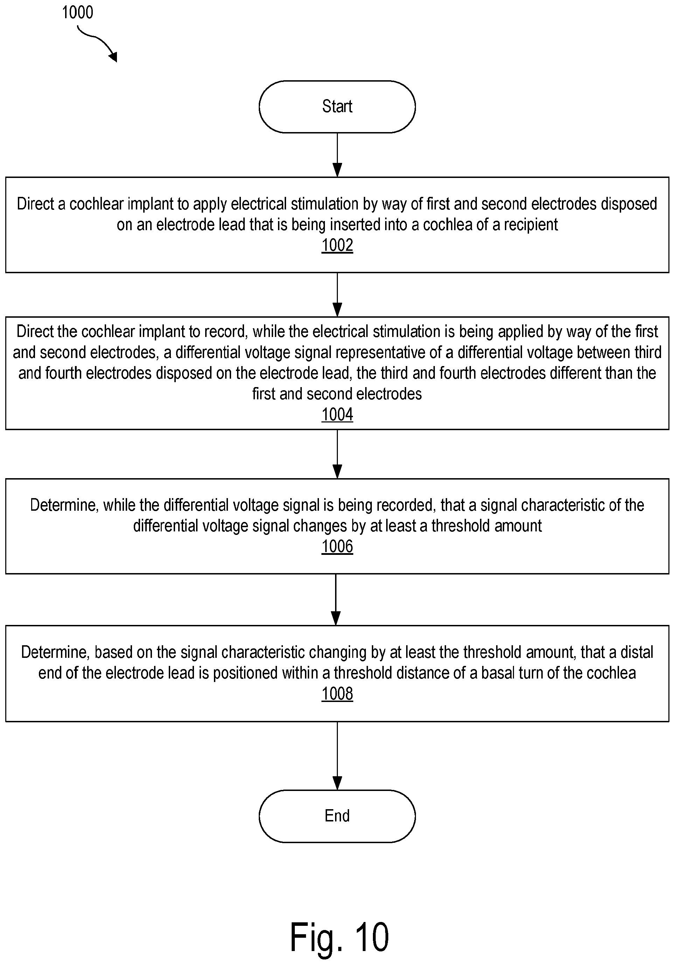

FIG. 10 illustrates another exemplary method according to principles described herein. The operations shown in FIG. 10 may be performed by system 300 and/or any implementation thereof. While FIG. 10 illustrates exemplary operations according to one embodiment, other embodiments may omit, add to, reorder, and/or modify any of the operations shown in FIG. 10.

In operation 1002, an insertion management system directs cochlear implant to apply electrical stimulation by way of first and second electrodes disposed on an electrode lead that is being inserted into a cochlea of a recipient. The first and second electrodes may be any two of electrodes 112 described herein. Operation 1002 may be performed in any of the ways described herein.

In operation 1004, the insertion management system directs the cochlear implant to record, while the electrical stimulation is being applied by way of the first and second electrodes, a differential voltage signal representative of a differential voltage between third and fourth electrodes disposed on the electrode lead. The third and fourth electrodes are different than the first and second electrodes. Operation 1004 may be performed in any of the ways described herein.

In operation 1006, the insertion management system determines, while the differential voltage signal is being recorded, that a signal characteristic of the differential voltage signal changes by at least a threshold amount. Operation 1006 may be performed in any of the ways described herein.

In operation 1008, the insertion management system determines, based on the signal characteristic changing by at least the threshold amount, that a distal end 114 of the electrode lead is positioned within a threshold distance of a basal turn of the cochlea. Operation 1008 may be performed in any of the ways described herein.

In some examples, a non-transitory computer-readable medium storing computer-readable instructions may be provided in accordance with the principles described herein. The instructions, when executed by a processor of a computing device, may direct the processor and/or computing device to perform one or more operations, including one or more of the operations described herein. Such instructions may be stored and/or transmitted using any of a variety of known computer-readable media.

A non-transitory computer-readable medium as referred to herein may include any non-transitory storage medium that participates in providing data (e.g., instructions) that may be read and/or executed by a computing device (e.g., by a processor of a computing device). For example, a non-transitory computer-readable medium may include, but is not limited to, any combination of non-volatile storage media and/or volatile storage media. Exemplary non-volatile storage media include, but are not limited to, read-only memory, flash memory, a solid-state drive, a magnetic storage device (e.g. a hard disk, a floppy disk, magnetic tape, etc.), ferroelectric random-access memory ("RAM"), and an optical disc (e.g., a compact disc, a digital video disc, a Blu-ray disc, etc.). Exemplary volatile storage media include, but are not limited to, RAM (e.g., dynamic RAM).

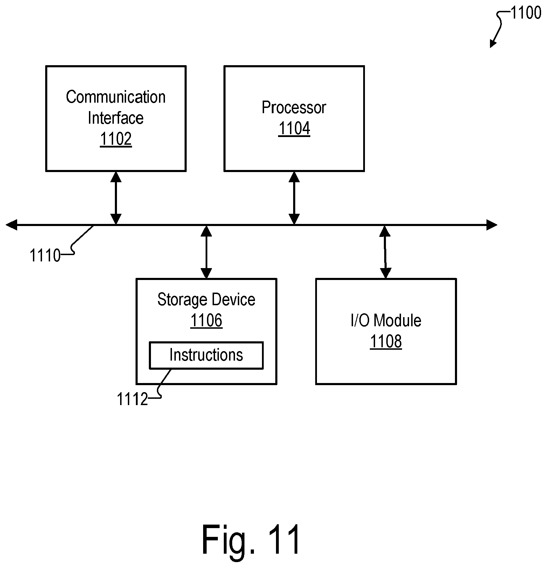

FIG. 11 illustrates an exemplary computing device 1100 that may be specifically configured to perform one or more of the processes described herein. As shown in FIG. 11, computing device 1100 may include a communication interface 1102, a processor 1104, a storage device 1106, and an input/output ("I/O") module 1108 communicatively connected one to another via a communication infrastructure 1110. While an exemplary computing device 1100 is shown in FIG. 11, the components illustrated in FIG. 11 are not intended to be limiting. Additional or alternative components may be used in other embodiments. Components of computing device 1100 shown in FIG. 11 will now be described in additional detail.

Communication interface 1102 may be configured to communicate with one or more computing devices. Examples of communication interface 1102 include, without limitation, a wired network interface (such as a network interface card), a wireless network interface (such as a wireless network interface card), a modem, an audio/video connection, and any other suitable interface.

Processor 1104 generally represents any type or form of processing unit capable of processing data and/or interpreting, executing, and/or directing execution of one or more of the instructions, processes, and/or operations described herein. Processor 1104 may perform operations by executing computer-executable instructions 1112 (e.g., an application, software, code, and/or other executable data instance) stored in storage device 1106.

Storage device 1106 may include one or more data storage media, devices, or configurations and may employ any type, form, and combination of data storage media and/or device. For example, storage device 1106 may include, but is not limited to, any combination of the non-volatile media and/or volatile media described herein. Electronic data, including data described herein, may be temporarily and/or permanently stored in storage device 1106. For example, data representative of computer-executable instructions 1112 configured to direct processor 1104 to perform any of the operations described herein may be stored within storage device 1106. In some examples, data may be arranged in one or more databases residing within storage device 1106.

I/O module 1108 may include one or more I/O modules configured to receive user input and provide user output. I/O module 1108 may include any hardware, firmware, software, or combination thereof supportive of input and output capabilities. For example, I/O module 1108 may include hardware and/or software for capturing user input, including, but not limited to, a keyboard or keypad, a touchscreen component (e.g., touchscreen display), a receiver (e.g., an RF or infrared receiver), motion sensors, and/or one or more input buttons.

I/O module 1108 may include one or more devices for presenting output to a user, including, but not limited to, a graphics engine, a display (e.g., a display screen), one or more output drivers (e.g., display drivers), one or more audio speakers, and one or more audio drivers. In certain embodiments, I/O module 1108 is configured to provide graphical data to a display for presentation to a user. The graphical data may be representative of one or more graphical user interfaces and/or any other graphical content as may serve a particular implementation.

In some examples, any of the systems, computing devices, and/or other components described herein may be implemented by computing device 1100. For example, storage facility 302 may be implemented by storage device 1106, and processing facility 304 may be implemented by processor 1104.

In the preceding description, various exemplary embodiments have been described with reference to the accompanying drawings. It will, however, be evident that various modifications and changes may be made thereto, and additional embodiments may be implemented, without departing from the scope of the invention as set forth in the claims that follow. For example, certain features of one embodiment described herein may be combined with or substituted for features of another embodiment described herein. The description and drawings are accordingly to be regarded in an illustrative rather than a restrictive sense.

## Claims

1. A system comprising:
a memory (302, 1106) storing instructions (306, 1112); and
a processor (304, 1104) communicatively coupled to the memory and configured to execute the instructions to:
direct a cochlear implant (108) to apply electrical stimulation by way of a first electrode (112-2) disposed on an electrode lead (110) that is being inserted into a cochlea (200) of a recipient;
direct the cochlear implant to record, while the electrical stimulation is being applied by way of the first electrode, a differential voltage signal representative of a differential voltage between a second electrode (112-1) and a third electrode (112-3) both disposed on the electrode lead, the second and third electrodes different than the first electrode;
determine, while the differential voltage signal is being recorded, that a signall characteristic of the differential voltage signal (702) changes by at least a threshold amount; and
determine, based on the signal characteristic changing by at least the threshold amount, that a distal end (114) of the electrode lead is positioned within a threshold distance of a basal turn (208) of the cochlea;
wherein the signal characteristic comprises a slope associated with the differential voltage signal, wherein the processor is further configured to execute the instructions to generate an impedance signal based on the differential voltage signal, and wherein the slope corresponds to the impedance signal.

2. The system of claim 1, wherein the memory (302, 1106) and processor (304, 1104) are implemented by at least one of a sound processor (104) in a cochlear implant system (100) and a computing device (402) configured to be communicatively coupled to the sound processor.

3. The system of claims 1, wherein the directing of the cochlear implant (108) to apply the electrical stimulation comprises directing the cochlear implant to apply a sequence of bipolar stimulation pulses by way of the first electrode (112-2) and a fourth electrode (112-4).

4. The system of claim 3, wherein as determined along a length of the electrode lead (110), the second electrode (112-1) is positioned closest to the distal end (114) of the electrode lead, and the first electrode (112-2) and the fourth electrode (112-4) are positioned adjacent one another in between the second and third electrodes (112-3).

5. The system of claim 1, wherein the processor (304, 1104) is further configured to execute the instructions (306, 1112) to plot, within an interface (800, 1108) displayed on a display device (404), a graph (700) associated with the differential voltage signal (702) in substantially real time as the differential voltage signal is being recorded.

6. The system of claim 7, wherein the processor (304, 1104) is further configured to execute the instructions (306, 1106) to present, within the interface (800, 1108) and in response to the determining that the distal end (114) is positioned within the threshold distance of the basal turn (208) of the cochlea (200), a notification that the distal end is positioned within the threshold distance of the basal turn of the cochlea.

7. The system of claim 1, wherein the processor (304, 1104) is further configured to execute the instructions (306, 1112) to present, within an interface (800, 1108) displayed on a display device (404), a progress indicator indicating a location of the electrode lead (110) within the cochlea (200) based on the signal characteristic.

8. The system of claim 1, wherein the processor (304, 1104) is further configured to execute the instructions to determine, based on the signal characteristic, a trajectory of insertion of the electrode lead (110) into the cochlea (200).

9. The system of claim 1, wherein the processor (304, 1104) is further configured to execute the instructions to determine, based on the signal characteristic changing by at least the threshold amount, a distance between a round window of the recipient and the basal turn (208).

10. The system of claim 1, wherein the processor (304, 1104) is further configured to execute the instructions (306, 1112) to:
direct, subsequent to determining that the distal end (114) is positioned within the threshold distance of the basal turn (208), the cochlear implant (108) to apply the electrical stimulation by way of a fourth electrode (112-4) disposed on the electrode lead (110);
direct the cochlear implant to record, while the electrical stimulation is being applied by way of the fourth electrode, an additional differential voltage signal representative of a differential voltage between a fifth electrode (112-5) and a sixth electrode (112-6) both disposed on the electrode lead, the fifth and sixth electrodes different than the fourth electrode;
determine, while the differential voltage signal between the fifth and sixth electrodes is being recorded, that a signal characteristic of the additional differential voltage signal changes by at least the threshold amount; and
determine, based on the signal characteristic of the additional differential voltage signal changing by at least the threshold amount, that at least one of the fourth, fifth, and sixth electrodes is positioned within the threshold distance of the basal turn.

## Patentansprüche

1. System mit:
einem Speicher (302, 1106), welcher Befehle (306, 1112) speichert; und
einem Prozessor (304, 1104), welcher kommunikativ mit dem Speicher gekoppelt ist und ausgebildet ist, um die Befehle auszuführen, um:
ein Cochlea-Implantat (108) zu veranlassen, elektrische Stimulation mittels einer ersten Elektrode (112-2), die auf einer Elektrodenzuleitung (110) angeordnet ist, anzuwenden, welche in eine Cochlea (200) eines Empfängers eingeführt wird;
das Cochlea-Implantat zu veranlassen, während die elektrische Stimulation mittels der ersten Elektrode angewandt wird, ein Differenzspannungssignal aufzuzeichnen, welches repräsentativ für eine Differenzspannung zwischen einer zweiten Elektrode (112-1) und einer dritten Elektrode (112-3) ist, die beide auf der Elektrodenzuleitung angeordnet sind und sich von der ersten Elektrode unterscheiden;
festzustellen, während das Differenzspannungssignal aufgezeichnet wird, dass sich eine Signalcharakteristik des Differenzspannungssignals (702) um mindestens einen Schwellwertbetrag ändert; und
basierend auf der sich um mindestens den Schwellwertbetrag ändernden Signalcharakteristik festzustellen, dass ein distales Ende (114) der Elektrodenzuleitung innerhalb eines Schwellwertabstands zu einer Basalwindung (208) der Cochlea angeordnet ist;
wobei die Signalcharakteristik eine mit dem Differenzspannungssignal assoziierte Flanke aufweist, wobei der Prozessor ferner ausgebildet ist, um die Befehle auszuführen, um ein Impedanzsignal basierend auf dem Differenzspannungssignal zu erzeugen, und wobei die Flanke dem Impedanzsignal entspricht.

2. System gemäß Anspruch 1, wobei der Speicher (302, 1106) und der Prozessor (304, 1104) mittels mindestens eines Schallprozessors (104) in einem Cochlea-Implantatsystem (100) und einer Berechnungsvorrichtung (402) implementiert sind, die ausgebildet ist, um mit dem Schallprozessor kommunikativ gekoppelt zu sein.

3. System gemäß Anspruch 1, wobei beim Veranlassen des Cochlea-Implantats (108) zum Anwenden der elektrischen Stimulation das Cochlea-Implantat veranlasst wird, eine Sequenz von bipolaren Stimulationspulsen mittels der ersten Elektrode (112-2) und einer vierten Elektrode (112-4) anzuwenden.

4. System gemäß Anspruch 3, wobei, wie entlang einer Länge der Elektrodenzuleitung (110) festgestellt wird, die zweite Elektrode (112-1) am nächsten zu dem distalen Ende (114) der Elektrodenzuleitung angeordnet ist und die erste Elektrode (112-2) und die vierte Elektrode (112-4) benachbart zueinander zwischen der zweiten und der dritten Elektrode (112-3) angeordnet sind.

5. System gemäß Anspruch 1, wobei der Prozessor (304, 1104) ferner ausgebildet ist, um die Befehle (306, 1112) auszuführen, um innerhalb einer auf einer Anzeigevorrichtung (404) angezeigten Schnittstelle (800, 1108) einen Graphen (700), der mit dem Differenzspannungssignal (702) assoziiert ist, im Wesentlichen in Echtzeit darzustellen, während das Differenzspannungssignal aufgezeichnet wird.

6. System gemäß Anspruch 5, wobei der Prozessor (304, 1104) ferner ausgebildet ist, um die Befehle (306, 1106) auszuführen, um innerhalb der Schnittstelle (800, 1108) als Reaktion darauf, dass festgestellt wird, dass das distale Ende (114) innerhalb des Schwellwertabstands zu der Basalwindung (208) der Cochlea (200) angeordnet ist, eine Benachrichtigung zu präsentieren, dass das distale Ende innerhalb des Schwellwertabstands zu der Basalwindung der Cochlea angeordnet ist.

7. System gemäß Anspruch 1, wobei der Prozessor (304, 1104) ferner ausgebildet ist, um die Befehle (306, 1112) auszuführen, um innerhalb einer auf einer Anzeigevorrichtung (404) angezeigten Schnittstelle (800, 1108) einen Fortschrittsindikator zu präsentieren, welcher einen Ort der Elektrodenzuleitung (110) innerhalb der Cochlea (200) basierend auf der Signalcharakteristik wiedergibt.

8. System gemäß Anspruch 1, wobei der Prozessor (304, 1104) ferner ausgebildet ist, um die Befehle auszuführen, um basierend auf der Signalcharakteristik eine Einführtrajektorie der Elektrodenzuleitung (110) in die Cochlea (200) zu bestimmen.

9. System gemäß Anspruch 1, wobei der Prozessor (304, 1104) ferner ausgebildet ist, um die Befehle auszuführen, um basierend auf der Änderung der Signalcharakteristik um mindestens den Schwellwertbetrag einen Abstand zwischen einem runden Fenster des Empfängers und der Basalwindung (208) festzustellen.

10. System gemäß Anspruch 1, wobei der Prozessor (304, 1104) ferner ausgebildet ist, um die Befehle (306, 1112) auszuführen, um:
nach dem Feststellen, dass das distale Ende (114) innerhalb des Schwellwertabstands zu der Basalwindung (208) angeordnet ist, das Cochlea-Implantat (108) zu veranlassen, mittels einer vierten Elektrode, die auf der Elektrodenzuleitung (110) angeordnet ist, elektrische Stimulation anzuwenden;
das Cochlea-Implantat zu veranlassen, während die elektrische Stimulation mittels der vierten Elektrode angewandt wird, ein zusätzliches Differenzspannungssignal aufzuzeichnen, welches repräsentativ für eine Differenzspannung zwischen einer fünften Elektrode (112-5) und einer sechsten Elektrode (112-6) ist, die beide auf der Elektrodenzuleitung angeordnet sind und sich von der vierten Elektrode unterscheiden;
während das Differenzspannungssignal zwischen der fünften und sechsten Elektrode aufgezeichnet wird, festzustellen, dass sich eine Signalcharakteristik des zusätzlichen Differenzspannungssignals um mindestens den Schwellwertbetrag ändert; und
basierend auf dem Ändern des zusätzlichen Differenzspannungssignals um mindestens den Schwellwertbetrag festzustellen, dass mindestens eine der vierten, fünften und sechsten Elektrode innerhalb des Schwellwertabstands zu der Basalwindung angeordnet ist.

## Revendications

1. Système comprenant :
une mémoire (302, 1106) stockant des instructions (306, 1112) ; et
un processeur (304, 1104) couplé en communication avec la mémoire et configuré pour exécuter les instructions pour :
ordonner à un implant cochléaire (108) d'appliquer une stimulation électrique au moyen d'une première électrode (112-2) disposée sur un porte-électrodes (110) qui est en cours d'insertion dans une cochlée (200) d'un receveur ;
ordonner à l'implant cochléaire d'enregistrer, pendant que la stimulation électrique est appliquée au moyen de la première électrode, un signal de tension différentielle représentatif d'une tension différentielle entre une deuxième électrode (112-1) et une troisième électrode (112-3) toutes les deux disposées sur le porte-électrodes, les deuxième et troisième électrodes étant différentes de la première électrode ;
déterminer, pendant que le signal de tension différentielle est enregistré, qu'une caractéristique de signal du signal de tension différentielle (702) change d'au moins une quantité seuil ; et
déterminer, sur la base de la caractéristique de signal changeant d'au moins la quantité seuil, qu'une extrémité distale (114) du porte-électrodes est positionnée dans les limites d'une distance seuil d'une spire basale (208) de la cochlée ;
dans lequel la caractéristique de signal comprend une pente associée au signal de tension différentielle, dans lequel le processeur est en outre configuré pour exécuter les instructions afin de générer un signal d'impédance sur la base du signal de tension différentielle, et dans lequel la pente correspond au signal d'impédance.

2. Système selon la revendication 1, dans lequel la mémoire (302, 1106) et le processeur (304, 1104) sont mis en œuvre par un processeur de sons (104) dans un système d'implant cochléaire (100) et/ou un dispositif informatique (402) configuré pour être couplé en communication au processeur de sons.

3. Système selon la revendication 1, dans lequel le fait d'ordonner à l'implant cochléaire (108) d'appliquer la stimulation électrique comprend le fait d'ordonner à l'implant cochléaire d'appliquer une séquence d'impulsions de stimulation bipolaires au moyen de la première électrode (112-2) et d'une quatrième électrode (112-4).

4. Système selon la revendication 3, dans lequel, comme déterminé sur une longueur du porte-électrodes (110), la deuxième électrode (112-1) est positionnée le plus près de l'extrémité distale (114) du porte-électrodes, et la première électrode (112-2) et la quatrième électrode (112-4) sont positionnées de manière adjacente l'une à l'autre entre les deuxième et troisième électrodes (112-3).

5. Système selon la revendication 1, dans lequel le processeur (304, 1104) est en outre configuré pour exécuter les instructions (306, 1112) pour tracer, à l'intérieur d'une interface (800, 1108) affichée sur un dispositif d'affichage (404), un graphique (700) associé au signal de tension différentielle (702) sensiblement en temps réel à mesure que le signal de tension différentielle est enregistré.

6. Système selon la revendication 7, dans lequel le processeur (304, 1104) est en outre configuré pour exécuter les instructions (306, 1106) pour présenter, à l'intérieur de l'interface (800, 1108) et en réponse à la détermination que l'extrémité distale (114) est positionnée dans les limites de la distance seuil de la spire basale (208) de la cochlée (200), une notification selon laquelle l'extrémité distale est positionnée dans les limites de la distance seuil de la spire basale de la cochlée.

7. Système selon la revendication 1, dans lequel le processeur (304, 1104) est en outre configuré pour exécuter les instructions (306, 1112) pour présenter, à l'intérieur d'une interface (800, 1108) affichée sur un dispositif d'affichage (404), un indicateur de progression indiquant un emplacement du porte-électrodes (110) à l'intérieur de la cochlée (200) sur la base de caractéristique de signal.

8. Système selon la revendication 1, dans lequel le processeur (304, 1104) est en outre configuré pour exécuter les instructions pour déterminer, sur la base de la caractéristique de signal, une trajectoire d'insertion du porte-électrodes (110) dans la cochlée (200).

9. Système selon la revendication 1, dans lequel le processeur (304, 1104) est en outre configuré pour exécuter les instructions pour déterminer, sur la base de la caractéristique de signal changeant d'au moins la quantité seuil, une distance entre une fenêtre ronde du receveur et la spire basale (208).

10. Système selon la revendication 1, dans lequel le processeur (304, 1104) est en outre configuré pour exécuter les instructions (306, 1112) pour :
ordonner, après avoir déterminé que l'extrémité distale (114) est positionnée dans les limites de la distance seuil de la spire basale (208), à l'implant cochléaire (108) d'appliquer la stimulation électrique au moyen d'une quatrième électrode (112-4) disposée sur le porte-électrodes (110) ;
ordonner à l'implant cochléaire d'enregistrer, pendant que la stimulation électrique est appliquée au moyen de la quatrième électrode, un signal de tension différentielle supplémentaire représentatif d'une tension différentielle entre une cinquième électrode (112-5) et une sixième électrode (112-6) toutes les deux disposées sur le porte-électrodes, les cinquième et sixième électrodes étant différentes de la quatrième électrode ;
déterminer, pendant que le signal de tension différentielle entre les cinquième et sixième électrodes est enregistré, qu'une caractéristique de signal de tension différentielle supplémentaire change d'au moins la quantité seuil ; et
déterminer, sur la base de la caractéristique de signal de tension différentielle supplémentaire qui change d'au moins la quantité seuil, qu'au moins une des quatrième, cinquième et sixième électrodes est positionnée dans les limites de la distance seuil de la spire basale.
